# EUROPEAN PATENT SPECIFICATION

(11) **EP 3 795 690 B1**
(45) Date of publication and mention of the grant of the patent: **19.11.2025**
(21) Application number: 19803477.9
(22) Date of filing: 19.04.2019
(51) Int. Cl.: A23D 9/02, C11B 3/16, C11B 7/00, C12N 1/14, C12P 7/6434, A23L 33/12, C11B 3/00

(54) **METHOD FOR ADJUSTING COMPOSITION OF FATTY ACID COMPOSITIONS IN DHA MICROBIAL OIL**
VERFAHREN ZUR ANPASSUNG DER ZUSAMMENSETZUNG VON FETTSÄUREZUSAMMENSETZUNGEN IN DHA-MIKROBIELLEM ÖL
PROCÉDÉ DE RÉGLAGE DE LA COMPOSITION DE COMPOSITIONS D'ACIDES GRAS DANS DE L'HUILE MICROBIENNE DE DHA

(30) Priority: 17.05.2018 CN 201810475952; 06.11.2018 CN 201811314254
(43) Date of publication of application: 24.03.2021
(73) Proprietor: Liang, Yun, Changsha, Hunan 410208 (CN)
(72) Inventor: QU, Hanpeng, Changsha, Hunan 410208 (CN); CAO, Sheng, Changsha, Hunan 410208 (CN); WANG, Shenjian, Changsha, Hunan 410208 (CN)
(74) Representative: Vitina, Maruta
(86) International application number: PCT/CN2019/083394
(87) International publication number: WO 2019/218839

(56) References cited:
- WO-A1-2016/059540
- CN-A- 1 648 233
- CN-A- 101 519 676
- CN-A- 105 349 588
- CN-A- 107 746 746
- CN-A- 109 371 071
- US-A1- 2010 239 533
- US-A1- 2017 356 018
- IIDA I ET AL: "Improvement of docosahexaenoic acid production in a culture of Thraustochytrium aureum by medium optimization", JOURNAL OF FERMENTATION AND BIOENGINEERING, SOCIETY OF FERMENTATION TECHNOLOGY, JP, vol. 81, no. 1, 1 January 1996 (1996-01-01), pages 76 - 78, XP002327281, ISSN: 0922-338X, DOI: 10.1016/0922-338X(96)83125-4
- TING YEN HUANG ET AL: "A fermentation strategy for producing docosahexaenoic acid in Aurantiochytrium limacinum SR21 and increasing C22:6 proportions in total fatty acid", BIORESOURCE TECHNOLOGY, vol. 123, 27 July 2012 (2012-07-27), AMSTERDAM, NL, pages 8 - 14, XP055582377, ISSN: 0960-8524, DOI: 10.1016/j.biortech.2012.07.068
- SINGHASUWAN SOMRUETHAI ET AL: "Carbon-to-nitrogen ratio affects the biomass composition and the fatty acid profile of heterotrophically grownChlorellasp. TISTR 8990 for biodiesel production", JOURNAL OF BIOTECHNOLOGY, ELSEVIER, AMSTERDAM NL, vol. 216, 20 October 2015 (2015-10-20), pages 169 - 177, XP029323787, ISSN: 0168-1656, DOI: 10.1016/J.JBIOTEC.2015.10.003

## Description

### TECHNICAL FIELD

The present disclosure relates to microbial fermentation, and more particularly to a method for adjusting a fatty acid composition in a DHA-containing microbial oil.

### BACKGROUND

Microbial oils, also named single cell oils are produced by oleaginous microorganisms such as yeasts, fungi and microalgae using carbon and nitrogen sources, and trace elements.

Oleaginous microorganisms are sources that are abundant and can grow under various culture conditions. It is promising in terms of industrial production of microbial oils. Microbial oil includes high levels of polyunsaturated fatty acids, for example, docosahexaenoic acid (DHA) and arachidonic acid (ARA), which are essential fatty acids for human to maintain important physiological functions.

A lack of ARA and DHA may cause permanent mental retardation and visual impairment in infants, and pruritus, dry eyes and distraction in class for children aged 6-12. The ARA and DHA are also effective in the prevention and treatment of hypertension, hyperlipidemia, diabetes and viral infection.

DHA and ARA products commercially available are mainly extracted from deep-sea fish oil, with unstable composition of polyunsaturated fatty acids. Due to the limitation on the source of raw materials, the manufacture of such products is costly and low-yield, and the realization of industrial production is impossible. Therefore, microbe oil has become an important resource to produce high-value fatty acids, such as linolenic acid (GLA), ARA, eicosapentaenoic acid (EPA) and DHA.

Nevertheless, recent studies show that excessive EPA has adverse effects on the growth and metabolism of fetuses and infants. Thus, the content of EPA in the editable microbial oil for pregnant women and infants should be restricted.

Microbes such as *Ukenella*, *Schizochytrium*, *Thraustochytrium*, *Cryptodinium* and yeast are commonly-used microorganisms for producing DHA oils because they can produce the microbial oil with high amounts of DHAs. However, in the prior art, the DHA microbial oils, produced by fermentation of microorganisms such as *Ukenella*, *Schizochytrium, Thraustochytrium*, *Cryptodinium* and yeast, have a relatively low content of DHA, and a high content of EPA and harmful fatty acids such as myristic acid, lauric acid and erucic acid, which hinders the use of microbial oils. CN 101 519 676 A discloses a method for production of DHA by fermentation of *Schizochytrium limacinum* wherein temperature is differentially controlled in the growth stage and in the oil accumulation stage. The microbial oil obtained at the end of the fermentation comprises up to 54% DHA, up to 0.3% EPA, up to 0.1% lauric acid and up to 4.5% myristic acid.

### SUMMARY

The object of the present disclosure is to provide a method for adjusting a fatty acid composition in a DHA-containing microbial oil to solve the problem that DHA microbe oils have a high content of harmful fatty acids and EPA and a low content of DHA. The method produces a DHA-containing microbial oil through a fermentation by microorganisms such as *Ukenella*, *Schizochytrium*, *Thraustochytrium*, *Cryptodinium* and yeast, and adjusts a fatty acid composition in the DHA-containing microbial oil so as to produce a microbial oil having high DHA content and a low content of harmful fatty acids.

The invention is set out in the appended claims.

The above-mentioned technical solutions improve a yield of a microbial oil, and the microbial oil has a high content of DHA, and a low content of EPA and harmful fatty acids such as myristic acid, lauric acid and erucic acid. The microbial oil is clear and transparent at -10-5°C.

### DETAILED DESCRIPTION OF EMBODIMENTS

It should be noted that endpoints and values within ranges disclosed herein are only exemplary, and are intended to include any values close to these values. Any possible combination of numerical values within the range to form one or more new ranges should be considered to be expressly disclosed in this disclosure.

In examples and comparative examples of the present invention, all fermentation strains to produce DHA-containing microbial oils are *Schizochytrium.* All strains and various culture supplies used are commercially available, and all reagents and detection methods involved are implemented in accordance with national standards.

### Example 1

1) Production of strains: Original strains were inoculated and cultured in a shake flask containing a sterilized and cooled culture medium.

A formula of the culture medium was: 4.5 wt% of glucose, 3.2 wt% of sodium glutamate, 0.62 wt% of yeast extract, 1.8 wt% of sodium chloride, 0.7 wt% of potassium dihydrogen phosphate, 0.55 wt% of magnesium sulfate, 0.03 wt% of calcium chloride, 0.1 wt% of trace elements (such as nickel sulfate, copper sulfate, sodium molybdate, manganese chloride, cobalt chloride, zinc sulfate and ferrous sulfate), and 0.1 wt% of vitamins (such as B2, B6 and B12).

Flasks were incubated on a shaker at 28±1°C for 36-44 hours at a speed of 200 rpm. The strains were then transferred to a primary seed tank upon the formation of mycelium.

2) Primary inoculum: The strains were inoculated and cultured in the primary seed tank containing a sterilized and cooled culture medium to obtain a primary inoculum.

A formula of the culture medium was: 3 wt% of glucose, 0.83 wt% of yeast powder, 0.62 wt% of sodium glutamate, 0.62 wt% of yeast extract, 0.62 wt% of sodium chloride, 0.7 wt% of potassium dihydrogen phosphate, 0.52 wt% of magnesium sulfate, 0.02 wt% of calcium chloride, 0.02 wt% of sodium bicarbonate, 0.93 wt% of sodium sulfate, 0.10 wt% of ammonium sulfate, 0.08 wt% of potassium chloride, 0.10 wt% of trace elements (such as nickel sulfate, copper sulfate, sodium molybdate, manganese chloride, cobalt chloride, zinc sulfate and ferrous sulfate), and 0.10 wt% of vitamins (such as B2, B6 and B12), and 0.03 wt% of epoxy silicon ether.

The primary seed tank was incubated at 30±2°C for 18-24 hours at a speed of 90 rpm. A ventilation was at 0.50 vvm.

3) Secondary inoculum: 4 wt% of the primary inoculum was inoculated and cultured in a secondary seed tank containing a sterilized and cooled culture medium to obtain a secondary inoculum.

A formula of the culture medium was: 5 wt% of glucose, 2.0 wt% of sodium glutamate, 1.0 wt% of yeast extract, 0.14 wt% of sodium chloride, 0.16 wt% of potassium dihydrogen phosphate, 0.50 wt% of magnesium sulfate, 0.02 wt% of calcium chloride, 0.02 wt% of sodium bicarbonate, 0.91 wt% of sodium sulfate, 0.10 wt% of ammonium sulfate, 0.08 wt% of potassium chloride, 0.09 wt% of trace elements (such as nickel sulfate, copper sulfate, sodium molybdate, manganese chloride, cobalt chloride, zinc sulfate and ferrous sulfate), 0.09 wt% of vitamins (such as B2, B6 and B12), and 0.36 wt% of epoxy silicon ether.

The secondary seed tank was incubated at 30±2°C for 14-16 hours at a speed of 90 rpm.

4) Fermentation: 10% the secondary inoculum was inoculated into a fermentor containing a fermentation medium.

A formula of the fermentation medium was: 5 wt% of glucose, 2.5 wt% of sodium glutamate, 1.0 wt% of yeast extract, 0.29 wt% of sodium chloride, 0.28 wt% of potassium dihydrogen phosphate, 0.66 wt% of magnesium sulfate, 0.03 wt% of calcium chloride, 0.02 wt% of sodium bicarbonate, 0.58 wt% of sodium sulfate, 0.13 wt% of ammonium sulfate, 0.11 wt% of potassium chloride, 0.13 wt% of trace elements (such as nickel sulfate, copper sulfate, sodium molybdate, manganese chloride, cobalt chloride, zinc sulfate and ferrous sulfate), 0.13 wt% of vitamins (such as B2, B6 and B12), and 0.03 wt% of epoxy silicon ether.

The fermentor was incubated at 30°C for 80 hours at a speed of 90 rpm. A ventilation was at 0.50 vvm. After 80 hours, the fermentor was incubated at 22°C. By addition of 50% glucose aqueous solution and 60% sodium glutamate aqueous solution, a content of glucose and sodium glutamate in the fermentation medium may be adjusted to control a carbon-to-nitrogen ratio. Table 1 showed, during 5 days of fermentation, the adjustment of contents of glucose and sodium glutamate in the fermentation medium and pH of the fermentation medium.

**Table 1**

| | | | | | |
|---|---|---|---|---|---|
| Time (hours) | 0-16 | 17-55 | 56-80 | 81-95 | 96-120 |
| Glucose (g/100mL) | 4-6 | 3-4 | 2-3 | 0.5-1 | < 0.5 |
| Sodium glutamate (g/100mL) | 2-3 | 1.5-2 | 1-1.2 | 0.75-1 | 0.5-0.75 |
| pH | without adjustment | 6.2-6.5 | 6.5-6.7 | 6.7-7.0 | 6.7-7.0 |

5) Extraction: After the fermentation, the mycelium was dehydrated with a three-phase centrifuge to separate from water, and then the dehydrated mycelium was further dehydrated with 95% ethanol and was extracted with hexane to obtain a crude oil.

6) Water washing and degumming: The crude oil was added with pure water which was 10% by weight of the crude oil. Then the oil was heated to 85°C and stirred at 80 rpm for 20 min. The oil stood for 2 hours and was separated from water.

7) Acid refining: The oil was heated to 75°C, added with citric acid which was 4‰ by weight of the oil, and stirred at 80 rpm for 40 min. Then the oil was added with hot water at 85°C which was 10% by weight of the oil, and stirred for 20 min. The oil was stood for 3 hours and was separated from water.

8) Alkali refining: The oil was heated to 45°C and added with alkali solution according to an acid value of the oil. (The addition=7.13×10⁻⁴×acid value×oil weight). The oil was added with 40% aqueous sodium hydroxide solution and stirred at 80 rpm for 50 min. Then the oil was heated to 80°C and added with pure water at 85°C which was 5% by weight of the oil, stirred at 80 rpm for 15 min. The oil was centrifuged by a two-phase centrifuge to remove saponins.

9) Dehydration: The oil was heated to 80°C and dehydrated at -0.1 MPa for 35 min.

10) Ambient-temperature winterization: The dehydrated oil was naturally cooled to 25°C for nucleation. The ambient-temperature winterization lasted for 20 hours.

11) Filtration: The winterized oil was filtered by a plate and frame filter press at a pressure of 0.3 MPa. A filter medium was industrial filter cloth.

12) Second dehydration: The filtered oil was heated to 80°C and dehydrated at -0.1 MPa for 35 min.

13) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 14°C, the oil was reheated at a rate of 1.5°C/h for 4.5 hours for crystal growth. After that, the oil was cooled at a rate of 1.5°C/h to -5°C, and the oil was kept at this temperature. The low-temperature winterization lasted for 70 hours.

14) Second filtration: The winterized oil was filtered by a plate and frame filter press at a pressure of 0.2 MPa. A filter medium was industrial filter cloth.

15) Decolorization: The oil was decolorized by activated carbon and activated clay for 70 min. The activated carbon was 1.5% by weight of the oil, and the activated clay was 1.5% by weight of the oil.

16) Deodorization: The decolorized oil was deodorized at 175±2°C for 4 hours. A steam pressure was maintained at 0.2-0.3 MPa. A vacuum degree is at 50 Pa, and a steam consumption was controlled at about 5% by weight of the oil. The oil was cooled and the vacuum was broken to obtain a product oil.

### Example 2

1) Production of strains: Original strains were inoculated and cultured in a shake flask containing a sterilized and cooled culture medium.

A formula of the culture medium was: 4.5 wt% of glucose, 3.2 wt% of sodium glutamate, 0.62 wt% of yeast extract, 1.8 wt% of sodium chloride, 0.7 wt% of potassium dihydrogen phosphate, 0.55 wt% of magnesium sulfate, 0.03 wt% of calcium chloride, 0.1 wt% of trace elements such as nickel sulfate, copper sulfate, sodium molybdate, manganese chloride, cobalt chloride, zinc sulfate and ferrous sulfate), and 0.1 wt% of vitamins (such as B2, B6 and B12).

Flasks were incubated on a shaker at 28±1°C for 36-44 hours at a speed of 200 rpm. The strains were then transferred to a primary seed tank upon the formation of mycelium.

2) Primary inoculum: The strains were inoculated and cultured in the primary seed tank containing a sterilized and cooled culture medium to obtain a primary inoculum.

A formula of the culture medium was: 3 wt% of glucose, 0.83 wt% of yeast powder, 0.62 wt% of sodium glutamate, 0.62 wt% of yeast extract, 0.62 wt% of sodium chloride, 0.7 wt% of potassium dihydrogen phosphate, 0.52 wt% of magnesium sulfate, 0.02 wt% of calcium chloride, 0.02 wt% of sodium bicarbonate, 0.93 wt% of sodium sulfate, 0.10 wt% of ammonium sulfate, 0.08 wt% of potassium chloride, 0.10 wt% of trace elements (such as nickel sulfate, copper sulfate, sodium molybdate, manganese chloride, cobalt chloride, zinc sulfate and ferrous sulfate), and 0.10 wt% of vitamins (such as B2, B6 and B12), and 0.03 wt% of epoxy silicon ether.

The primary seed tank was incubated at 30±2°C for 18-24 hours at a speed of 90 rpm. A ventilation was at 0.50 vvm.

3) Secondary inoculum: 4 wt% of the primary inoculum was inoculated and cultured in a secondary seed tank containing a sterilized and cooled culture medium to obtain a secondary inoculum.

A formula of the culture medium was: 5 wt% of glucose, 2.0 wt% of sodium glutamate, 1.0 wt% of yeast extract, 0.14 wt% of sodium chloride, 0.16 wt% of potassium dihydrogen phosphate, 0.50 wt% of magnesium sulfate, 0.02 wt% of calcium chloride, 0.02 wt% of sodium bicarbonate, 0.91 wt% of sodium sulfate, 0.10 wt% of ammonium sulfate, 0.08 wt% of potassium chloride, 0.09 wt% of trace elements (such as nickel sulfate, copper sulfate, sodium molybdate, manganese chloride, cobalt chloride, zinc sulfate and ferrous sulfate), 0.09 wt% of vitamins (such as B2, B6 and B12), and 0.36 wt% of epoxy silicon ether.

The secondary seed tank was incubated at 30±2°C for 14-16 hours at a speed of 90 rpm.

4) Fermentation: 10% (v/v) of the secondary inoculum was inoculated into a fermentor containing a fermentation medium.

A formula of the fermentation medium was: 5 wt% of glucose, 2.5 wt% of sodium glutamate, 1.0 wt% of yeast extract, 0.29 wt% of sodium chloride, 0.28 wt% of potassium dihydrogen phosphate, 0.66 wt% of magnesium sulfate, 0.03 wt% of calcium chloride, 0.02 wt% of sodium bicarbonate, 0.58 wt% of sodium sulfate, 0.13 wt% of ammonium sulfate, 0.11 wt% of potassium chloride, 0.13 wt% of trace elements (such as nickel sulfate, copper sulfate, sodium molybdate, manganese chloride, cobalt chloride, zinc sulfate and ferrous sulfate), 0.13 wt% of vitamins (such as B2, B6 and B12), and 0.03 wt% of epoxy silicon ether.

The fermentor was incubated at 29°C for 80 hours at a speed of 90 rpm. A ventilation was at 0.5 vvm. After 80 hours, the fermentor was incubated at 19°C. By addition of 50% glucose aqueous solution and 60% sodium glutamate aqueous solution, a content of glucose and sodium glutamate in the fermentation medium may be adjusted to control a carbon-to-nitrogen ratio. Table 2 showed, during 5 days of fermentation, the adjustment of contents of glucose and sodium glutamate in the fermentation medium and pH of the fermentation medium.

**Table 2**

| | | | | | |
|---|---|---|---|---|---|
| Time (hours) | 0-16 | 17-55 | 56-80 | 81-95 | 96-120 |
| Glucose (g/100mL) | 2-4 | 2-3 | 1-2 | 0.5-1 | < 0.5 |
| Sodium glutamate (g/100mL) | 1-2 | 1-1.5 | 1-1.2 | 0.75-1 | 0.5-0.75 |
| pH | without adjustment | 6.2-6.5 | 6.5-6.7 | 6.7-7.0 | 6.7-7.0 |

5) Extraction: After the fermentation, the mycelium was dehydrated with a three-phase centrifuge to separate from water, and then the dehydrated mycelium was further dehydrated with 95% ethanol and was extracted with hexane to obtain a crude oil.

6) Water washing and degumming: The crude oil was added with pure water which was 10% by weight of the crude oil. Then the oil was heated to 85°C and stirred at 80 rpm for 20 min. The oil was stood for 2 hours and was separated from water.

7) Acid refining: The oil was heated to 75°C, added with citric acid which was 4‰ by weight of the oil, and stirred at 80 rpm for 40 min. Then the oil was added with hot water at 85°C which was 10% by weight of the oil, and stirred for 20 min. The oil was stood for 3 hours and was separated from water.

8) Alkali refining: The oil was heated to 45°C and added with alkali solution according to an acid value of the oil. (The addition=7.13×10⁻⁴× the acid value×the oil weight). The oil was added with 40% aqueous sodium hydroxide solution and stirred at 80 rpm for 50 min. Then the oil was heated to 80°C and added with pure water at 85°C which was 5% by weight of the oil, stirred at 80 rpm for 15 min. The oil was centrifuged by a two-phase centrifuge to remove saponins.

9) Dehydration: The oil was heated to 70°C and dehydrated at -0.1 MPa for 35 min.

10) Ambient-temperature winterization: The dehydrated oil was naturally cooled to 20°C for nucleation. The ambient-temperature winterization lasted for 16 hours.

11) Filtration: The winterized oil was filtered by a plate and frame filter press at a pressure of 0.3 MPa. A filter medium was industrial filter cloth.

12) Second dehydration: The filtered oil was heated to 80°C and dehydrated at -0.1 MPa for 35 min.

13) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 15°C, the oil was reheated at a rate of 1°C/h for 4.5 hours for crystal growth. After that, the oil was cooled at a rate of 2°C/h to 1°C, and the oil was kept at this temperature. The low-temperature winterization lasted for 48 hours.

14) Second filtration: The winterized oil was filtered by a plate and frame filter press at a pressure of 0.2 MPa. A filter medium was industrial filter cloth.

15) Decolorization: The oil was decolorized by activated carbon and activated clay for 70 min. The activated carbon was 1.5% by weight of the oil, and the activated clay was 1.5% by weight of the oil.

16) Deodorization: The decolorized oil was deodorized at 175±2°C for 4 hours. A steam pressure was maintained at 0.2-0.3 MPa. A vacuum degree is at 50Pa, and a steam consumption was controlled at about 5% by weight of the oil. The oil was cooled and the vacuum was broken to obtain a product oil.

### Example 3

1) Production of strains: Original strains were inoculated and cultured in a shake flask containing a sterilized and cooled culture medium.

A formula of the culture medium was: 4.5 wt% of glucose, 3.2 wt% of sodium glutamate, 0.62 wt% of yeast extract, 1.8 wt% of sodium chloride, 0.7 wt% of potassium dihydrogen phosphate, 0.55 wt% of magnesium sulfate, 0.03 wt% of calcium chloride, 0.1 wt% of trace elements (such as nickel sulfate, copper sulfate, sodium molybdate, manganese chloride, cobalt chloride, zinc sulfate and ferrous sulfate), and 0.1 wt% of vitamins (such as B2, B6 and B12).

Flasks were incubated on a shaker at 28±1°C for 36-44 hours at a speed of 200 rpm. The strains were then transferred to a primary seed tank upon the formation of mycelium.

2) Primary inoculum: The strains were inoculated and cultured in the primary seed tank containing a sterilized and cooled culture medium to obtain a primary inoculum.

A formula of the culture medium was: 3 wt% of glucose, 0.83 wt% of yeast powder, 0.62 wt% of sodium glutamate, 0.62 wt% of yeast extract, 0.62 wt% of sodium chloride, 0.7 wt% of potassium dihydrogen phosphate, 0.52 wt% of magnesium sulfate, 0.02 wt% of calcium chloride, 0.02 wt% of sodium bicarbonate, 0.93 wt% of sodium sulfate, 0.10 wt% of ammonium sulfate, 0.08 wt% of potassium chloride, 0.10 wt% of trace elements (such as nickel sulfate, copper sulfate, sodium molybdate, manganese chloride, cobalt chloride, zinc sulfate and ferrous sulfate), and 0.10 wt% of vitamins (such as B2, B6 and B12), and 0.03 wt% of epoxy silicon ether.

The primary seed tank was incubated at 30±2°C for 18-24 hours at a speed of 90 rpm. A ventilation was at 0.50 vvm.

3) Secondary inoculum: 4 wt% of the primary inoculum was inoculated and cultured in a secondary seed tank containing a sterilized and cooled culture medium to obtain a secondary inoculum.

A formula of the culture medium was: 5 wt% of glucose, 2.0 wt% of sodium glutamate, 1.0 wt% of yeast extract, 0.14 wt% of sodium chloride, 0.16 wt% of potassium dihydrogen phosphate, 0.50 wt% of magnesium sulfate, 0.02 wt% of calcium chloride, 0.02 wt% of sodium bicarbonate, 0.91 wt% of sodium sulfate, 0.10 wt% of ammonium sulfate, 0.08 wt% of potassium chloride, 0.09 wt% of trace elements (such as nickel sulfate, copper sulfate, sodium molybdate, manganese chloride, cobalt chloride, zinc sulfate and ferrous sulfate), 0.09 wt% of vitamins (such as B2, B6 and B12), and 0.36 wt% of epoxy silicon ether.

The secondary seed tank was incubated at 30±2°C for 14-16 hours at a speed of 90 rpm.

4) Fermentation: 10% (v/v) of the secondary inoculum was inoculated into a fermentor containing a fermentation medium.

A formula of the fermentation medium was: 5 wt% of glucose, 2.5 wt% of sodium glutamate, 1.0 wt% of yeast extract, 0.29 wt% of sodium chloride, 0.28 wt% of potassium dihydrogen phosphate, 0.66 wt% of magnesium sulfate, 0.03 wt% of calcium chloride, 0.02 wt% of sodium bicarbonate, 0.58 wt% of sodium sulfate, 0.13 wt% of ammonium sulfate, 0.11 wt% of potassium chloride, 0.13 wt% of trace elements (such as nickel sulfate, copper sulfate, sodium molybdate, manganese chloride, cobalt chloride, zinc sulfate and ferrous sulfate), 0.13 wt% of vitamins (such as B2, B6 and B12), and 0.03 wt% of epoxy silicon ether.

The fermentor was incubated at 32°C for 80 hours at a speed of 90 rpm. After 80 hours, the fermentor was incubated at 28°C. A ventilation was at 0.50 vvm. By addition of 50% glucose aqueous solution and 60% sodium glutamate aqueous solution, a content of glucose and sodium glutamate in the fermentation medium may be adjusted to control a carbon-to-nitrogen ratio. Table 3 showed, during 5 days of fermentation, the adjustment of contents of glucose and sodium glutamate in the fermentation medium and pH of the fermentation medium.

**Table 3**

| | | | | | |
|---|---|---|---|---|---|
| Time (hours) | 0-16 | 17-55 | 56-80 | 81-95 | 96-120 |
| Glucose (g/100mL) | 6-8 | 5-6 | 4-5 | 0.5-1 | < 0.5 |
| Sodium glutamate (g/100mL) | 1-5 | 1-3 | 1-2 | 0.75-1 | 0.5-0.75 |
| pH | without adjustment | 6.2-6.5 | 6.5-6.7 | 6.7-7.0 | 7.0-7.4 |

5) Extraction: After the fermentation, the mycelium was dehydrated with a three-phase centrifuge to separate from water, and then the dehydrated mycelium was further dehydrated with 95% ethanol and was extracted with hexane to obtain a crude oil.

6) Water washing and degumming: The crude oil was added with pure water which was 10% by weight of the crude oil. Then the oil was heated to 85°C and stirred at 80 rpm for 20 min. The oil was stood for 2 hours and was separated from water.

7) Acid refining: The oil was heated to 75°C and added with citric acid which was 4‰ by weight of the oil, stirred at 80 rpm for 40 min. Then the oil was added with hot water at 85°C which was 10% by weight of the oil, stirred for 20 min. The oil was settled for 3 hours thereafter and was separated from water.

8) Alkali refining: The oil was heated to 45°C and added with 40% aqueous sodium hydroxide solution. The amount of the addition of the aqueous sodium hydroxide solution was in accordance with an acid value of the oil (the amount of the addition=7.13×10⁻⁴× the acid value × weight of the oil). The oil was stirred at 80 rpm for 50 min. Then the oil was heated to 80°C and added with pure water at 85°C which was 5% by weight of the oil, stirred at 80 rpm for 15 min. The oil was centrifuged by a two-phase centrifuge to remove saponins.

9) Dehydration: The oil was heated to 90°C and dehydrated at -0.1 MPa for 35 min.

10) Ambient-temperature winterization: The dehydrated oil was naturally cooled to 30°C to for nucleation. The ambient-temperature winterization lasted for 24 hours.

11) Filtration: The winterized oil was filtered by a plate and frame filter press at a pressure of 0.3 MPa. A filter medium was industrial filter cloth.

12) Second dehydration: The filtered oil was heated to 85°C and dehydrated at -0.1 MPa for 35 min.

13) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 14°C, the oil was reheated at a rate of 2°C/h for 5 hours for crystal growth. After that, the oil was cooled at a rate of 1°C/h to -10°C. The crystal growth lasted for at least 16 hours. The low-temperature winterization lasted for 70 hours.

14) Second filtration: The winterized oil was filtered by a plate and frame filter press at a pressure of 0.2 MPa. A filter medium was industrial filter cloth.

15) Decolorization: The oil was decolorized by activated carbon and activated clay for 70 min. The activated carbon was 1.5% by weight of the oil, and the activated clay was 1.5% by weight of the oil.

16) Deodorization: The decolorized oil was deodorized at 175±2°C for 4 hours. A steam pressure was maintained at 0.2-0.3 MPa. A vacuum degree is at 50 Pa, and a steam consumption was controlled at about 5% by weight of the oil. The oil was cooled and the vacuum was broken to obtain a product oil.

### Comparative Example

A method used herein was basically the same as that used in Example 1 except that a formula of a fermentation medium was: 5 wt% of glucose, 2.5 wt% of sodium glutamate, and 1.0 wt% of yeast extract (a carbon-to-nitrogen ratio of was 10.7:1); strains was thermostatically cultured at 20°C for 5 days; and the oil was rapidly cooled to 0°C for 48 hours for crystal growth.

### Results and Comparison

Oil products in Examples 1-3 and Comparative example were detected by gas chromatography for analysis of fatty acid composition. Weight percentage of individual fatty acids in respective oil products were shown in Table 4.

**Table 4**

| Name | Example 1 | Example 2 | Example 3 | Comparative Example |
|---|---|---|---|---|
| Lauric acid (C12:0) | 0.105 | 0.124 | 0.119 | 1.47 |
| Myristic acid (C14:0) | 0.822 | 0.793 | 0.851 | 1.89 |
| Myristic acid (C14:1) | 0.024 | 0.031 | 0.035 | 1.62 |
| Palmitic acid (C16:0) | 14.806 | 17.975 | 19.112 | 27.92 |
| Palmitoleic acid C16:1 | 0.51 | 0.493 | 0.572 | 0.11 |
| Stearic acid (C18:0) | 1.01 | 1.21 | 1.17 | 17.49 |
| Oleic acid (C18:1, n-9) | 2.18 | 2.31 | 2.28 | 0.22 |
| Linoleic acid (C18:2, n-6) | 0.83 | 0.79 | 0.91 | 0.13 |
| γ-linolenic acid (C18:3, n-6) | 2.12 | 2.37 | 2.29 | 0.23 |
| ARA (C20:4, n-6) | 0.147 | 0.159 | 0.143 | 0.12 |
| Arachidic acid (C22:0) | 1.64 | 2.612 | 2.722 | 4.51 |
| EPA (C20:5, n-3) | 0.93 | 0.89 | 0.96 | 0.27 |
| DPA (C22:5, n-6) | 13.45 | 15.68 | 15.71 | 0.24 |
| DHA (C22:6, n-3) | 55.71 | 48.76 | 47.25 | 35.31 |
| Tetracosanoic acid (C24:0) | 1.23 | 1.32 | 1.25 | 3.29 |
| Erucic acid (C22:1, n-9) | 0.141 | 0.163 | 0.171 | 0.133 |
| Other fatty acids | 4.331 | 4.324 | 4.452 | 5.04 |
| DHA:EPA | 59.90 | 54.79 | 49.22 | 34.06 |
| Unsaturated fatty acids: Saturated fatty acids | 3.17 | 2.52 | 2.36 | 0.58 |

Table 4 showed that the oil produced by the present method had a higher content of DHA and a lower content of harmful fatty acids. In addition, the oil produced by the method according to the present disclosure had better low-temperature solidification performance.

The resulting microbial oil has a high content of DHA and a low content of EPA and harmful fatty acids, and a good low-temperature solidification performance. The microbial oil can be used to produce infant formula, especially milk powder. In addition, the microbial oil can also be applied in nutraceuticals for those who need DHA for health care, and health food and ordinary food as a supplement of daily intake.

## Claims

1. A method for adjusting a fatty acid composition in a docosahexaenoic acid (DHA) microbial oil, **characterized by** comprising:
during 0-16 hours of a fermentation of *Schizochytrium,* controlling a content of glucose at 2-8 g/100 mL and a content of sodium glutamate at 1-5 g/100 mL, and controlling a culture temperature at 29-32°C and a carbon-to-nitrogen ratio in a medium at 3-20:1;
during 17-55 hours of the fermentation, controlling the content of glucose at 2-6 g/100 mL and the content of sodium glutamate at 1-3 g/100 mL, and controlling a culture temperature at 29-32°C and the carbon-to-nitrogen ratio in the medium at 3-20:1;
during 56-80 hours of the fermentation, controlling the content of glucose at 1-5 g/100 mL and the content of sodium glutamate at 1-2 g/100 mL, and controlling a culture temperature at 29-32°C and the carbon-to-nitrogen ratio in the medium at 3-20:1;
during 81-95 hours of the fermentation, controlling the content of glucose at 0.5-1.0 g/100 mL and the content of sodium glutamate at 0.75-1.0 g/100 mL, and controlling a culture temperature at 19-28°C and the carbon-to-nitrogen ratio in the medium at 1-15:1; and
after 95 hours of the fermentation, controlling the content of glucose to less than 0.5 g/100 mL and the content of sodium glutamate at 0.5-0.75 g/100 mL, and controlling a culture temperature at 19-28°C and the carbon-to-nitrogen ratio in the medium at 1-15:1.

2. The method according to claim 1, **characterized in that** a refining process of the microbial oil comprises:
heating the microbial oil to 70-90°C to dewater the microbial oil;
cooling the microbial oil to 20-30°C;
performing an ambient-temperature winterization on the microbial oil for 16-24 hours;
filtering the microbial oil, and dewatering the microbial oil; and
cooling the microbial oil according to a set program to -10-1°C to perform a low-temperature winterization on the microbial oil for 48-90 hours.

## Patentansprüche

1. Verfahren zum Einstellen einer Fettsäurezusammensetzung in einem mikrobiellen Öl mit Docosahexaensäure (DHA), **dadurch gekennzeichnet, dass** es umfasst:
während 0-16 Stunden einer Fermentation von *Schizochytrium,* Steuern eines Gehalts an Glucose auf 2-8 g/100 mL und eines Gehalts an Natriumglutamat auf 1-5 g/100 mL, und Steuern einer Kulturtemperatur auf 29-32°C und eines Kohlenstoff-Stickstoff-Verhältnisses in einem Medium auf 3-20:1;
während 17-55 Stunden der Fermentation, Steuern des Gehalts an Glucose auf 2-6 g/100 mL und des Gehalts an Natriumglutamat auf 1-3 g/100 mL, und Steuern einer Kulturtemperatur auf 29-32°C und des Kohlenstoff-Stickstoff-Verhältnisses in dem Medium auf 3-20:1;
während 56-80 Stunden der Fermentation, Steuern des Gehalts an Glucose auf 1-5 g/100 mL und des Gehalts an Natriumglutamat auf 1-2 g/100 mL, und Steuern einer Kulturtemperatur auf 29-32°C und des Kohlenstoff-Stickstoff-Verhältnisses in dem Medium auf 3-20:1;
während 81-95 Stunden der Fermentation, Steuern des Gehalts an Glucose auf 0,5-1,0 g/100 mL und des Gehalts an Natriumglutamat auf 0,75-1,0 g/100 mL, und Steuern einer Kulturtemperatur auf 19-28°C und des Kohlenstoff-Stickstoff-Verhältnisses in dem Medium auf 1-15:1; und
nach 95 Stunden der Fermentation, Steuern des Gehalts an Glucose auf weniger als 0,5 g/100 mL und des Gehalts an Natriumglutamat auf 0,5-0,75 g/100 mL, und Steuern einer Kulturtemperatur auf 19-28°C und des Kohlenstoff-Stickstoff-Verhältnisses in dem Medium auf 1-15:1.

2. Verfahren nach Anspruch 1, **dadurch gekennzeichnet, dass** ein Raffinationsprozess des mikrobiellen Öls umfasst:
Erhitzen des mikrobiellen Öls auf 70 bis 90 °C, um das mikrobielle Öl zu entwässern;
Abkühlen des mikrobiellen Öls auf 20 bis 30 °C;
Durchführen einer Umgebungstemperatur-Winterisierung an dem mikrobiellen Öl für 16 bis 24 Stunden;
Filtrieren des mikrobiellen Öls, und Entwässern des mikrobiellen Öls; und
Abkühlen des mikrobiellen Öls gemäß einem festgelegten Programm auf -10 bis 1 °C, um eine Niedertemperatur-Winterisierung an dem mikrobiellen Öl für 48 bis 90 Stunden durchzuführen.

## Revendications

1. Procédé d'ajustement d'une composition en acides gras dans une huile microbienne à base d'acide docosahexaénoïque (DHA), **caractérisé en ce qu'**il comprend :
pendant 0 à 16 heures de fermentation de *Schizochytrium,* contrôler une teneur en glucose à 2-8 g/100 mL et une teneur en glutamate de sodium à 1-5 g/100 mL, et contrôler une température de culture à 29-32°C et un rapport carbone/azote dans un milieu à 3-20:1 ;
pendant 17 à 55 heures de fermentation, contrôler la teneur en glucose à 2-6 g/100 mL et la teneur en glutamate de sodium à 1-3 g/100 mL, et contrôler une température de culture à 29-32°C et le rapport carbone/azote dans le milieu à 3-20:1 ;
pendant 56 à 80 heures de fermentation, contrôler la teneur en glucose à 1-5 g/100 mL et la teneur en glutamate de sodium à 1-2 g/100 mL, et contrôler une température de culture à 29-32°C et le rapport carbone/azote dans le milieu à 3-20:1 ;
pendant 81 à 95 heures de fermentation, contrôler la teneur en glucose à 0,5-1,0 g/100 mL et la teneur en glutamate de sodium à 0,75-1,0 g/100 mL, et contrôler la température de culture à 19-28°C et le rapport carbone/azote dans le milieu à 1-15:1 ; et
après 95 heures de fermentation, contrôler la teneur en glucose à moins de 0,5 g/100 mL et la teneur en glutamate de sodium à 0,5-0,75 g/100 mL, et contrôler une température de culture à 19-28°C et le rapport carbone/azote dans le milieu à 1-15:1.

2. Procédé selon la revendication 1, **caractérisé en ce qu'**un processus de raffinage de l'huile microbienne comprend les étapes suivantes :
chauffer l'huile microbienne à 70-90°C pour déshydrater l'huile microbienne ;
refroidir l'huile microbienne à 20-30°C ;
effectuer une hivérisation à température ambiante sur l'huile microbienne pendant 16 à 24 heures ;
filtrer l'huile microbienne et déshydrater l'huile microbienne ; et
refroidir l'huile microbienne selon un programme défini à -10-1°C pour effectuer une hivérisation à basse température sur l'huile microbienne pendant 48 à 90 heures.
